# EUROPÄISCHE PATENTSCHRIFT

(11) **EP 2 379 483 B1**
(45) Veröffentlichungstag und Bekanntmachung des Hinweises auf die Patenterteilung: **26.06.2013**
(21) Anmeldenummer: 09768101.9
(22) Anmeldetag: 15.12.2009
(51) Int. Cl.: C07C 67/58, C07C 69/00, C07C 69/80

(54) **VERFAHREN ZUR AUFARBEITUNG EINES ROHEN ESTERS**
METHOD FOR REGENERATION OF A RAW ESTER
PROCÉDÉ DE TRAITEMENT D'UN ESTER BRUT

(30) Priorität: 16.12.2008 EP 08171800
(43) Veröffentlichungstag der Anmeldung: 26.10.2011
(73) Patentinhaber: BASF SE, 67056 Ludwigshafen (DE)
(72) Erfinder: FRIESE, Katrin, 68199 Mannheim (DE); DISTELDORF, Walter, 67157 Wachenheim (DE); PETERS, Jarren, 68163 Mannheim (DE); GOLFIER, Günther, 67227 Frankenthal (DE)
(86) Internationale Anmeldenummer: PCT/EP2009/067178
(87) Internationale Veröffentlichungsnummer: WO 2010/076193

(56) Entgegenhaltungen:
- EP-A1- 0 439 722
- DE-A1- 1 945 359
- DE-A1- 2 330 435

## Beschreibung

Die Erfindung betrifft ein Verfahren zur Aufarbeitung eines rohen Esters einer Veresterungsreaktion, die mit einem metallhaltigen Veresterungskatalysator katalysiert ist.

Ester der Phthalsäure, Adipinsäure, Sebacinsäure oder Maleinsäure finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Vielfach werden Metallverbindungen als Katalysatoren verwendet, wie die Alkoholate, Carboxylate und Chelatverbindungen von Titan, Zirkonium, Zinn, Zink und Aluminium.

Obgleich die katalytischen Eigenschaften dieser metallhaltigen Katalysatoren zufriedenstellend sind, bereitet die Entfernung der Katalysatorrückstände aus den Veresterungsprodukten Schwierigkeiten. Zur Reinigung versetzt man die rohen Ester in der Regel zunächst zur Neutralisation von nicht oder unvollständig umgesetzter Säure (Partialester) mit Alkalihydroxiden und entfernt die freien Alkohole durch Wasserdampfdestillation. Nach kurzer Vakuumdestillation zur Trocknung des Produkts werden dann die Katalysator-Rückstände durch Filtration entfernt. Da die Katalysator-Rückstände in der Regel von schleimiger, gelartiger Konsistenz sind, ist die Filtration meist nur unter Zuhilfenahme von Filtrierhilfsmitteln, wie z. B. Aktivkohle, Holzmehl oder Kieselgur, möglich. Dennoch ist eine derartige Filtration noch mit schwerwiegenden Nachteilen verbunden: Es werden lange Filtrationszeiten benötigt und die Ausbeute an Ester wird verringert, weil im Filterkuchen große Mengen Produkt festgehalten werden.

Aus der DE 194 53 59 geht ein Verfahren zur Aufarbeitung von rohen Weichmachern hervor, das die folgenden aufeinanderfolgenden Schritte aufweist: (i) die Restsäure im Roh-Weichmacher wird mit alkalischen Stoffen (z. B. 25 %ige Natronlauge) neutralisiert; (ii) die freien Alkohole im Roh-Weichmacher werden durch WasserdampfDestillation entfernt; (iii) das Produkt wird auf Temperaturen abgekühlt, die unter dem Siedepunkt des Wassers beim jeweiligen Druck liegen; (iv) es werden mindestens 0,5 Gewichtsprozent Wasser, bezogen auf das aufzuarbeitende Produkt, zugesetzt; (v) das Gemisch aus Wasser und aufzuarbeitendem Produkt wird mindestens 15 Minuten bei Temperaturen, die unter der Siedetemperatur des Wassers beim jeweiligen Druck liegen, intensiv gerührt; (vi) das zugesetzte Wasser wird durch Vakuumdestillation entfernt; (vii) der Weichmacher wird filtriert.

Bei Zugabe der Natronlauge unter den angegebenen Bedingungen verdampft sofort ein wesentlicher Teil des mit der wässrigen Lauge zugeführten Wassers, so dass festes Natriumhydroxid ausfällt. Festes Natriumhydroxid reagiert wesentlich langsamer als gelöstes NaOH. Außerdem führt die Ausfällung zu Ablagerungen an Rohrleitungen und Behältern, die häufiges Reinigen erforderlich machen.

Die DE 23 30 435 beschreibt ein Verfahren zur Aufarbeitung von rohen Estern, bei dem man den 140-250 °C heißen rohen Ester bei vermindertem Druck gleichzeitig mit wässrigen Lösungen von Alkali- oder Erdalkalihydroxid neutralisiert und durch Versetzen mit Wasser bei vermindertem Druck einer Wasserdampfdestillation unterwirft, anschließend trocknet und die gebildeten festen Bestandteile abfiltriert. Der Druck und die Geschwindigkeit der Wasserzugabe sollen so geregelt werden, dass das zugegebene Wasser rasch verdampft.

Unter den Verfahrensbedingungen, bei denen zugegebenes Wasser sofort verdampft, kann festes Alkali- oder Erdalkalihydroxid ausfallen, was zu den vorstehend beschriebenen Nachteilen führt. Da festes Hydroxid wesentlich langsamer reagiert, sind zur vollständigen Neutralisation bisweilen hohe Basenüberschüsse erforderlich.

Die EP 1 300 388 offenbart ein Verfahren zur Herstellung von Carbonsäureestem, wobei der überschüssige Alkohol nach der Veresterungsreaktion entfernt, der so erhaltene Rohester durch Basenzugabe neutralisiert und anschließend filtriert wird. Der Alkohol wird durch mindestens eine Wasserdampfdestillation abgetrennt und die Basenzugabe erfolgt während einer Wasserdampfdestillation. Die Lauge soll unten in das Reaktionsgemisch eingedüst werden. Durch die hohe Temperatur verdampft das Wasser. Durch geringe Zudosierungsgeschwindigkeiten der Lauge sollen Nebenreaktionen, wie beispielsweise die Verseifung der Ester, gering gehalten werden. Dies hat aber den Nachteil langer Neutralisationszeiten bzw. geringer Durchsätze.

Die US 5,434,294 beschreibt ein Verfahren zur Titanat-katalysierten Herstellung von Weichmacherestern. Das Produkt wird mit wässriger Base behandelt und dann unter Zuhilfenahme eines Filtrierhilfsmittels wie Bleicherde, Hydrotalcit oder Magnesiumsilikat, filtriert.

Die WO 97/11048 veranschaulicht die Herstellung von gemischten Phthalsäureestern. Die Umsetzung eines Phthalsäurehalbesters mit einem Polyethylenglykolmonomethylether wird mit Tetraisopropyl-Titan katalysiert. Nach beendeter Umsetzung wird tropfenweise Natriumbicarbonatlösung zugegeben. Nach dem Abkühlen gibt man 2 % Wasser zu, destilliert flüchtige Verbindungen, wie Wasser und Lösungsmittel, im Vakuum ab und filtriert.

Die DE 197 21 347 offenbart ein Verfahren zur Herstellung von Esterweichmachern, bei dem man eine Mischung von Säure oder Säureanhydrid und Alkohol zunächst bei 100 bis 160 °C unter Entfernung gegebenenfalls gebildeten Wassers miteinander reagieren lässt, die Reaktion unter Zusatz des Katalysators und durch Erhöhen der Temperatur bis auf 250 °C zu Ende führt, das Reaktionsgemisch mit einer wässrigen Alkali - oder Erdalkalihydroxid-Lösung umsetzt, darauf den überschüssigen Alkohol abtrennt, den zurückbleibenden Rohester trocknet und filtriert. Die alkalische Behandlung soll zweckmäßigerweise unmittelbar im Anschluss an den Veresterungsschritt ohne vorherige Abkühlung des Reaktionsgemisches erfolgen.

Der Erfindung liegt die Aufgabe zugrunde, ein Verfahren zur Aufarbeitung eines rohen Estergemisches anzugeben, das mit hohem Durchsatz in gut reproduzierbarer Weise zu Estern mit niedriger Säurezahl führt und bei dem die festen Katalysator-Rückstände in gut abfiltrierbarer Form anfallen.

Die Aufgabe wird gelöst durch ein Verfahren zur Aufarbeitung eines rohen Esters einer Veresterungsreaktion, die mit einem metallhaltigen Veresterungskatalysator katalysiert ist, bei dem man
a) den rohen Ester bei einer Temperatur T von mehr als 100 °C unter einem Druck p, der gleich oder größer ist als der Dampfdruck von Wasser bei der Temperatur T, mit einer wässrigen Base versetzt,
b) das Ester-Base-Gemisch entspannt und Wasser abdampft,
c) die erhaltene flüssige Phase unter Bildung einer Wasser-in-Öl-Emulsion mit Wasser versetzt,
d) aus der Emulsion Wasser abdestilliert und
e) den Ester filtriert.

Das erfindungsgemäße Verfahren umfasst mehrere Schritte: eine Neutralisation unter Druck mit anschließender Entspannung (Schritte a) und b)); eine Umbenetzungsagglomeration (Schritte c) und d)) und eine Filtration (Schritt e)).

Das Verfahren kann kontinuierlich durchgeführt werden, wobei die einzelnen Schritte in hintereinander geschalteten kontinuierlich betriebenen Apparaturen durchgeführt werden. Alternativ kann das Verfahren diskontinuierlich durchgeführt werden, wobei die einzelnen Schritte nacheinander in einer einzigen Apparatur, z. B. einem Rührbehälter, durchgeführt werden.

Zuerst wird der Veresterungskatalysator durch Zugabe einer wässrigen Base desaktiviert und ausgefällt. Gleichzeitig werden die bei der Veresterungsreaktion nicht umgesetzte Säure bzw. Partialester der Säure in Salze überführt. Es wurde festgestellt, dass eine ausreichend schnelle und vollständige Neutralisation erreicht wird, wenn die wässrige Base bei einer Temperatur T von mehr als 100 °C unter einem Druck p, der gleich oder größer ist als der Dampfdruck von Wasser bei der Temperatur T, zugegeben wird. Der rohe Ester, der nach der Veresterungsreaktion bzw. nach der Abtrennung von überschüssigem Alkohol vorliegt, weist im Allgemeinen eine erhöhte Temperatur auf. Er kann gegebenenfalls abgekühlt werden, jedoch nur soweit, dass seine Temperatur noch mehr als 100 °C beträgt. Die Zugabe der wässrigen Base erfolgt unter Druckbedingungen, unter denen das Wasser nicht spontan verdampft. Die Base steht daher für die Neutralisationsreaktion vollständig in gelöster, flüssiger Form zur Verfügung. Dies beschleunigt die Reaktion und erlaubt einen vollständigen Umsatz. Würde die wässrige Base bei geringerem Druck zugegeben werden, würde Wasser verdampfen und die gelöste Base als Feststoff ausfallen. Die feste Base stünde nicht oder nur mit deutlich geringerer Reaktionsgeschwindigkeit für die Neutralisation zur Verfügung. Im erfindungsgemäßen Verfahren kann die eingesetzte Menge an Base verringert werden, wodurch sich auch die Menge an zu entsorgendem Feststoff verringert. Die Bildung fester Ablagerungen an Behälterwänden oder Rohrleitungen bzw. Verstopfen der Rohrleitungen wird vermieden.

In der Regel weist der rohe Ester eine Temperatur T von 120 bis 185 °C auf. Der zugehörige Dampfdruck pᵥₐₚ von Wasser kann der nachstehenden Tabelle oder dem Fachmann bekannten Nachschlagewerken entnommen werden. Dem Fachmann ist bekannt, dass der Dampfdruck von Lösungsmitteln von gelösten Stoffen oder Mischungsphänomenen beeinflusst wird. Diese Einflüsse können vorliegend vernachlässigt werden. Für die Zwecke der vorliegenden Erfindung wird auf den Dampfdruck von reinem Wasser abgestellt.

**Tabelle: Dampfdruck von Wasser**

| T [°C] | pᵥₐₚ [bar] |
|---|---|
| 105 | 1.208 |
| 110 | 1.432 |
| 115 | 1.690 |
| 120 | 1.985 |
| 125 | 2.320 |
| 130 | 2.700 |
| 135 | 3.128 |
| 140 | 3.613 |
| 145 | 4.154 |
| 150 | 4.758 |
| 160 | 6.179 |
| 170 | 7.917 |
| 180 | 10.026 |
| 190 | 12.549 |
| 200 | 15.547 |

In der Regel ist der Druck p, bei dem der Schritt a) durchgeführt wird, höher als der Dampfdruck pᵥₐₚ bei der Temperatur T. Vorzugsweise beträgt der Druck p wenigstens das 1,1-fache von pᵥₐₚ, insbesondere wenigstens das 1,25-fache von pᵥₐₚ. Drücke von mehr als 25 bar sind technisch nur aufwendig zu realisieren und daher nicht bevorzugt.

Die Zugabe der wässrigen Base kann auf beliebige geeignete Weise erfolgen. Sie erfolgt vorzugsweise unterhalb der Flüssigkeitsoberfläche des rohen Esters. Hierfür eignen sich z. B. Lanzen oder Düsen, die an einem Behälterboden oder der Behälterwand vorgesehen sind. Das Gemisch wird dann intensiv durchmischt, z. B. mittels Rührer oder einer Umwälzpumpe.

Bei kontinuierlicher Durchführung führt man den Schritt a) zweckmäßigerweise durch, indem man die wässrige Base in einen Strom des rohen Esters einspritzt. Zur homogenen Einmischung der wässrigen Base führt man den gemischten Strom durch wenigstens einen Mischer. Hierbei kommen dynamische Mischer oder statische Mischer oder Kombinationen davon in Betracht. Statische Mischer sind bevorzugt. Die statischen Mischer lassen sich strömungsmechanisch in Turbulenz- und Laminarmischer unterteilen. Bei den Turbulenzmischern kommen sowohl freie turbulenzerzeugende Mischsysteme als auch solche mit Einbauten in Betracht. Zu den geeigneten statischen Mischern zählen Multiflux-Mischer, Wendelmischer, Wirbelmischer, Gittermischer, Sulzer-SMX-Mischer, Sulzer-SMV-Mischer und Kenics-Mischer. In einer geeigneten Ausführungsform ist der statische Mischer ein Rohr mit einer querschnittsverengenden Blende. Der Drucksprung hinter der Blende erzeugt eine Turbulenz, die zu einer ausreichenden Vermischung führt.

Die zugegebene Menge wässrige Base ist so bemessen, dass sie zur vollständigen Neutralisation der sauren Komponenten des rohen Esters ausreicht. In der Praxis wird ein mehr oder weniger großer Überschuss an Base eingesetzt. Die Gesamtmenge der sauren Komponenten des rohen Esters wird zweckmäßig durch die Säurezahl (in mg KOH/g) erfasst. Vorzugsweise bringt man mit der wässrigen Base 100 bis 300 % Neutralisationsäquivalente ein, bezogen auf die Säurezahl des rohen Esters, insbesondere 130 bis 220 %. Unter Neutralisationsäquivalent wird dabei die Menge Base verstanden, die die gleiche Zahl Protonen binden kann, wie 1 mg KOH. Mit anderen Worten verwendet man einen Basenüberschuss von bis zu 200 %, vorzugsweise 30 bis 120 %.

Als wässrige Base kommen Lösungen von Hydroxiden, Carbonaten, Hydrogencarbonaten von Alkalimetallen und Erdalkalimetallen in Betracht. Wässrige Alkalimetallhydroxidlösungen sind im Allgemeinen bevorzugt. Wässrige Natriumhydroxidlösung ist aufgrund ihrer leichten Verfügbarkeit besonders bevorzugt.

Die Konzentration der wässrigen Base ist an sich nicht kritisch, jedoch kann es beim Einsatz konzentrierter Alkalilösungen an der Einleitstelle der Base zur Hydrolyse der Ester kommen. Andererseits soll die Konzentration der wässrigen Base nicht zu niedrig sein, da das mit der wässrigen Base eingebrachte Wasser im nachfolgenden Schritt wieder entfernt werden muss. Daher sind wässrige Basen mäßiger bis geringer Konzentration bevorzugt, z. B. solche einer Konzentration von 0,5 bis 25 Gew.-%, insbesondere 1 bis 10 Gew.-%. Wässrige Natriumhydroxidlösung mit einer Konzentration von 1 bis 5 Gew.-% ist besonders bevorzugt.

Man hält das Ester-Base-Gemisch eine Haltezeit, z. B. 15 Sekunden bis 10 Minuten, vorzugsweise 30 Sekunden bis 5 Minuten, beim Druck p. Bei kontinuierlicher Verfahrensführung passiert das Gemisch während der Haltezeit z. B. eine Mischstrecke.

Im nachfolgenden Schritt wird das Ester-Base-Gemisch entspannt, z. B. auf einen Druck von weniger als 800 mbar, insbesondere weniger als 250 mbar, z. B. 50 bis 150 mbar. Auf diese Weise kann das mit der wässrigen Base eingebrachte Wasser entfernt werden, ohne den Rohester übermäßig thermisch zu belasten. Infolge der Entspannung trennt sich das Gemisch in eine flüssige Phase und eine Dampfphase. Mit der Dampfphase, die abgezogen wird, wird das mit der wässrigen Base eingeführte Wasser wieder entfernt. Neben dem mit der wässrigen Base eingeführten Wasser dampft bei dieser Behandlung gewöhnlich auch ein Teil des Restalkohols ab. Die Wasser und Alkohol enthaltenden Brüden können gesammelt und kondensiert werden und verworfen oder einer Wiederverwendung zugeführt werden.

Die flüssige Phase weist nach der Entspannung im Allgemeinen eine Temperatur von 130 bis 200 °C auf. Hierzu kann bei Bedarf die flüssige Phase erwärmt werden.

Die Art des Entspannungsgefäßes ist nicht kritisch. Z. B. kann man das Gemisch in einen Rührkessel entspannen, in dem eine weitere Behandlung der flüssigen Phase erfolgt.

Zur Vervollständigung des Abdampfens des Wassers ist es bevorzugt, die bei der Entspannung erhaltene flüssige Phase während einer Verweilzeit von z. B. 5 Minuten bis 1 Stunde, insbesondere 10 bis 40 Minuten, unter vermindertem Druck mechanisch zu bewegen. Hierzu eignen sich Rührer verschiedener Bauarten, wie z. B. ein Kreuzbalkenrührer.

Nach dem Schritt b) liegt der ausgefallene Feststoff, der im Wesentlichen aus Katalysator-Zersetzungsprodukten und Salzen von nicht umgesetzter Säure bzw. Partialestern mehrbasiger Säuren besteht, in fein verteilter, schwer filtrierbarer Form vor. Das erfindungsgemäße Verfahren sieht daher Maßnahmen vor, bei denen die feinen Teilchen zu größeren, leicht abtrennbaren Teilchen agglomeriert werden.

Hierzu versetzt man die flüssige Phase unter Bildung einer Wasser-in-Öl-Emulsion mit Wasser. Das Wasser wird als disperse Phase in Form feiner Tröpfchen in der flüssigen organischen Phase verteilt. Die feinen Feststoffteilchen wandern an die Grenzfläche zwischen Wassertröpfchen und umgebender organischer Phase. Bei der nachfolgenden Verdampfung des Wassers agglomerieren die feinen Teilchen und bilden grobe, gut abtrennbare Teilchen.

Damit sich eine eigene Wasserphase ausbildet, muss die zugegebene Wassermenge größer sein, als der Löslichkeit von Wasser in der organischen Phase entspricht. Die Wasserlöslichkeit in der organischen Phase hängt unter anderem vom Gehalt an nicht umgesetzten Alkohol ab, da der Alkohol als Lösungsvermittler wirkt. Je höher der Alkoholgehalt, umso mehr Wasser muss im Schritt c) zugesetzt werden. Bei üblichen Restalkoholgehalten von 1 bis 3 Gew.-% sind im Allgemeinen Mengen von 10 bis 60 g Wasser, vorzugsweise 20 bis 40 g, bezogen auf 1 kg rohen Ester, geeignet.

Die Wasserphase wird mit einem geeigneten Rührer oder Homogenisator in feine Tröpfchen zerteilt. Die erzeugten Wassertröpfchen weisen vorzugsweise eine mittlere Tröpfchengröße von weniger als 1000 µm auf. Als Rührer mit hoher spezifischer Rührleistung eignen sich beispielsweise Scheibenrührer. Alternativ kann man besonders bei kontinuierlicher Verfahrensführung eine Mischdüse verwenden, bei der über ein Dispergierventil Wasser direkt in den Rohesterstrom zugegeben wird.

Der Schritt c) erfolgt zweckmäßigerweise bei etwa Normaldruck.

Aus der so erzeugten Emulsion wird im nächsten Schritt das Wasser wieder abdestilliert. Vorzugsweise wird ein Blasensieden vermieden. Hierzu kann die Emulsion durch einen Verdampfer, z. B. einen Fallfilmverdampfer, geführt werden. Alternativ kann man die Emulsion unter vermindertem Druck mechanisch bewegen, z. B. rühren. Das Rühren erfolgt zweckmäßigerweise unter wenig scherenden Bedingungen. Übermäßiger Eintrag von Scherenergie könnte die noch labilen Agglomerate der festen Katalysator-rückstände wieder zu unerwünschten feinteiligen Partikeln zerteilen. Vorzugsweise destilliert man das Wasser bei einer Temperatur von 60 bis weniger als 100 °C und einem Druck von weniger als 500 mbar ab. Gewünschtenfalls kann man das Wasser auch in mehreren Schritten in aufeinander folgenden Rührbehältern abdestillieren, wobei im zweiten oder weiteren Schritt ein niedrigerer Druck und/oder eine höhere Temperatur als im vorhergehenden Schritt angewandt wird. Das Überführen von einem Rührbehälter in den darauffolgenden Rührbehälter erfolgt vorzugsweise unter wenig scherenden Bedingungen, z. B. durch freien Überlauf und nicht durch Umpumpen. Neben dem Emulsionswasser destilliert bei dieser Behandlung gewöhnlich auch ein Teil des Restalkohols ab. Die Wasser und Alkohol enthaltenden Brüden können gesammelt und kondensiert werden und verworfen oder einer Wiederverwendung zugeführt werden.

Nach dieser Behandlung liegt der Feststoff in gut filtrierbarer Form vor; es schlägt kein Feinanteil bei der Filtration durch. Die Anwendung von Filtrierhilfsmitteln ist nicht erforderlich; ihre Verwendung ist nicht bevorzugt. Zur Filtration des Esters eignen sich alle geeigneten Filter wie Kammerfilterpressen, Bandfilter, Kerzenfilter oder Tellerfilter. Zur kontinuierlichen Verfahrensführung eignen sich besonders Tellerfilter mit Zentrifugalkuchenabwurf. Der abgetrennte Feststoff wird verworfen.

Nach der Filtration kann der Ester verschiedenen Nachbehandlungen unterzogen werden, wie einer Dampfstrippung oder dergleichen.

Der im erfindungsgemäßen Verfahren eingesetzte rohe Ester entstammt einem üblichen Veresterungsverfahren. Derartige Verfahren sind dem Fachmann bekannt und in vielen Patentveröffentlichungen beschrieben. Dabei wird wenigstens eine Carbonsäure und/oder Carbonsäureanhydrid mit einem Alkohol oder Alkoholgemisch umgesetzt. Vielfach dient der Alkohol gleichzeitig als Schleppmittel für das bei der Umsetzung entstehende Reaktionswasser und wird daher im Überschuss eingesetzt. Vorzugsweise entfernt man vor dem Schritt a) aus dem rohen Ester die Hauptmenge des hier noch enthaltenen nicht umgesetzten Alkohols. Der Alkoholgehalt des im Schritt a) eingesetzten rohen Esters beträgt im Allgemeinen weniger als 5 Gew.-%, z. B. 1 bis 3 Gew.-%.

Im Veresterungsverfahren werden als Säurekomponente Carbonsäuren und/oder Carbonsäureanhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden. Die Säuren können aliphatisch, einschließlich carbocyclisch, heterocyclisch, gesättigt oder ungesättigt, sowie aromatisch, einschließlich heteroaromatisch, sein.

Zu den geeigneten Carbonsäuren zählen aliphatische Monocarbonsäuren mit wenigsten 5 Kohlenstoffatomen, insbesondere 5 bis 20 Kohlenstoffatomen, wie n-Pentansäure, 2-Methylbuttersäure, 3-Methylbuttersäure, 2-Methylpentansäure, 2-Ethylbuttersäure, n-Heptansäure, Isoheptansäuren, 2-Methylhexansäure, Cyclohexancarbonsäure, n-Octansäure, 2-Ethylhexansäure, Isooctansäuren, n-Nonansäure, 2-Methyloctansäure, Isononansäuren, n-Decansäure, Isodecansäuren, 2-Methylundecansäure, Isoundecansäure, Tricyclodecancarbonsäure und Isotridecansäure.

Weiter eignen sich aliphatische C₄-C₁₀-Dicarbonsäuren bzw. deren Anhydride, wie Maleinsäure, Fumarsäure, Maleinsäureanhydrid, Bernsteinsäure, Bernsteinsäureanhydrid, Adipinsäure, Korksäure, Trimethyladipinsäure, Azelainsäure, Decandisäure, Dodecandisäure, Brassylsäure. Beispiele für carbocyclische Verbindungen sind: Hexahydrophthalsäureanhydrid (Cyclohexan-1,2-dicarbonsäureanhydrid), Hexahydrophthalsäure (Cyclohexan-1,2-dicarbonsäure), Cyclohexan-1,4-dicarbonsäure, Cyclohex-4-en-1,2-dicarbonsäure, Cyclohexen-1,2-dicarbonsäureanhydrid, 4-Methylcyclohexan-1,2-dicarbonsäure, 4-Methylcyclohexan-1,2-dicarbonsäureanhydrid, 4-Methylcyclohex-4-en-1,2-dicarbonsäure, 4-Methylcyclohex-4-en-1,2-dicarbonsäureanhydrid.

Beispiele geeigneter aromatischer Dicarbonsäuren bzw. deren Anhydride sind: Phthalsäure, Phthalsäureanhydrid, Isophthalsäure, Therephthalsäure, oder Naphthalindicarbonsäuren und deren Anhydride.

Beispiele geeigneter aromatischer Tricarbonsäuren bzw. deren Anhydride sind Trimellitsäure, Trimellisäureanhydrid oder Trimesinsäure; Beispiele einer geeigneten aromatischen Tetracarbonsäure bzw. ihres Anhydrids sind Pyromellitsäure und Pyromellitsäureanhydrid.

Besonders bevorzugt wird Phthalsäureanhydrid oder Adipinsäure als Carbonsäurekomponente eingesetzt.

Es werden bevorzugt verzweigte oder lineare aliphatische Alkohole mit 4 bis 13 C-Atomen eingesetzt. Die Alkohole sind einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im Veresterungsverfahren eingesetzt werden, sind beispielsweise n-Butanol, Isobutanol, n-Octanol(1), n-Octanol(2), 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Z. B. können C₉/C₁₁-Alkoholgemische eingesetzt werden.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägem eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Process., Int. Ed. (1986) 65 (2. Sect. 1), Seite 31-33).

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess, mit anschließender Hydroformylierung und Hydrierung hergestellt werden.

Weiterhin eignen sich Alkylenglykolmonoether, insbesondere Ethylenglykolmonoether, z. B. Ethylenglykolmono-C₁-C₁₈-alkylether, wie Ethylenglykolmonomethylether, Ethylenglykolmonoethylether, Ethylenglykolpropylether, Ethylenglykolmonobutylether (2-Butoxy-ethanol) und deren Gemische; und Polyalkylenglykolmonoether insbesondere Polyethylenglykolmonoether, wie Polyethylenglykolmonomethylether.

Besonders bevorzugte Alkohohole sind 2-Ethylhexanol, 2-Propylheptanol, Isononanol-Isomerengemische, Decanol-Isomerengemische und C₉/C₁₁-Alkoholgemische sowie Ethylenglykolmonobutylether.

Geeigneterweise ist der Veresterungskatalysator unter Alkoholaten, Carboxylaten und Chelatverbindungen von Titan, Zirkonium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetrasec-butyltitanat, Tetraoctyltitanat, Tetra-(2-ethylhexyl)-titanat; Dialkyltitanate ((RO)₂TiO₂, worin R z.B. für iso-Propyl, n-Butyl, iso-Butyl steht), wie Isopropyl-n-butyltitanat; Titan-Acetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Di-n-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon-Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Alumini-umtris(ethylacetylacetonat). Insbesondere werden Isopropyl-n-butyltitanat, Tetra(isopropyl)orthotitanat oder Tetra(butyl)orthotitanat eingesetzt.

Die Katalysatorkonzentration beträgt im Allgemeinen 0,005 bis 1,0 Gew.-% bezogen auf das Reaktionsgemisch, insbesondere 0,01 bis 0,3 Gew.-%.

Der umzusetzende Alkohol, der als Schleppmittel dient, kann im stöchiometrischen Überschuss, bevorzugt 30 bis 200 %, besonders bevorzugt 50 bis 100 % der stöchiometrisch notwendigen Menge eingesetzt werden.

Die so hergestellten Ester aus mehrbasischen Carbonsäuren, wie beispielsweise Phthalsäure, Adipinsäure, Sebacinsäure, Maleinsäure, und aus Alkoholen, finden weite Anwendung in Lackharzen, als Bestandteile von Anstrichmitteln und insbesondere als Weichmacher für Kunststoffe. Spezielle Ester, die nach dem erfindungsgemäßen Verfahren aufgearbeitet werden können, sind Weichmacher für PVC, wie Dioctylphthalate, Diisononylphthalate, Diisodecylphthalate und Dipropylheptylphthalate; Weichmacher, beispielsweise für den Einsatz in Polyvinylbutyral, wie Dibutylglycoladipat, Dioctylazelat, Dioctyladipat, Dibutylsebacat, Di-(2-ethylhexyl)sebacat und Dioctylsebacat, sowie Dibutylglycolphthalat.

Die Erfindung wird durch die beigefügte Zeichnung und die folgenden Beispiele näher erläutert.

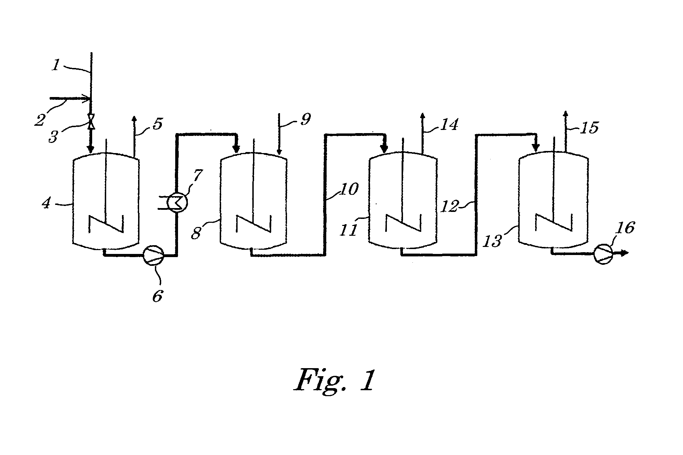

Fig. 1 zeigt eine zur Durchführung des erfindungsgemäßen Verfahrens geeignete Anlage. Über die Leitung 1 wird das rohe Estergemisch mit einer Temperatur von z. B. etwa 150 °C und einem Druck von 10 bar herangeführt. In den Esterstrom wird über die Leitung 2 wässrige Base, z. B. wässrige Natriumhydroxid- oder Kaliumhydroxidlösung dosiert. Das Gemisch passiert einen statischen Mischer (nicht dargestellt), um die wässrige Base homogen in den Rohesterstrom einzumischen. Um eine Verdampfung von Wasser zu verhindern, wird in der Rohrleitung ein Druck gehalten, der über dem Dampfdruck von Wasser bei der vorherrschenden Temperatur liegt. Die Verweilzeit in der Mischstrecke vor dem Ventil 3 beträgt z. B. 1 bis 2 min. Über das Ventil 3 wird der Rohesterstrom dann in den Rührkessel 4 entspannt. Der im Rührkessel 4 anfallende Brüden wird über die Leitung 5 abgeführt und kann kondensiert und gesammelt werden.

Über die Pumpe 6 und den Wärmetauscher 7 wird das Rohestergemisch in den Rührkessel 8 übergeführt. Über die Leitung 9 wird Wasser zugegeben. Unter den Druck - und Temperaturbedingungen im Kessel 8 (z. B. 80 °C, Normaldruck) verdampft das zugesetzte Wasser nicht sofort und wird durch Rühren als disperse Phase im Estergemisch in Form kleiner Tröpfchen verteilt. Der Rührer hat eine hohe spezifische Rührleistung und ist z. B. ein Scheibenrührer.

Die Emulsion gelangt über die Leitung 10 in den Rührkessel 11 und über die Leitung 12 in den Rührkessel 13. In den Rührkesseln 11 und 13 herrschen Druck- und Temperaturbedingungen (z. B. 80 °C und 100 mbar im Kessel 11; 80 °C und 50 mbar im Kessel 13), unter denen Wasser und gegebenenfalls freier Alkohol abdestillieren und über die Abzüge 14 bzw. 15 abgeführt werden. Am Ablauf des Kessels 13 liegen die Katalysator-Rückstände in gut filtrierbarer fester Form im Ester vor. Der Ester kann über die Pumpe 16 einer Filtrationseinheit (nicht dargestellt) zugeführt werden, in der der Feststoff abfiltriert wird.

### Beispiele

### Beispiel 1

Ein Strom von 6500 g/h rohes Diisononylphthalat (DINP) mit einer Säurezahl von 0,2 mg KOH/g und einem Alkoholgehalt von 2,4 Gew.-% wurde kontinuierlich aufgearbeitet.

Der DINP-Strom mit einer Temperatur von etwa 145 °C wurde unter einem Druck von 6 bar mit 174 g/h 1 %iger wässriger Natriumhydroxidlösung (entsprechend 90 % Überschuss, bezogen auf die Säurezahl des Rohesters) versetzt. Der gemischte Strom passierte eine Mischstrecke; die Verweilzeit in der Mischstrecke betrug etwa 1 min. Dann wurde der Strom in einen ersten Rührbehälter auf etwa 100 mbar entspannt. Die Verweilzeit im ersten Rührbehälter betrug etwa 0,5 h, während der das Gemisch mit einem dreistufigen Kreuzbalkenrührer bei 160 °C gerührt wurde.

Das Gemisch wurde in einen zweiten Rührbehälter umgepumpt und dabei auf etwa 80 °C gekühlt. Im zweiten Rührbehälter herrschte Umgebungsdruck. Man setzte 130 g/h Wasser (entsprechend 2 Gew.-%, bezogen auf den Rohesterstrom) zu. Die Verweilzeit im zweiten Rührbehälter betrug etwa 0,5 h, während der das Gemisch mit einem Scheibenrührer (spezifischer Leistungseintrag: 3 W/I) intensiv durchmischt wurde.

Die Emulsion wurde mit einer Temperatur von 80 °C in einen dritten Rührbehälter übergeführt. Im dritten Rührbehälter herrschte ein Druck von etwa 100 mbar. Die Verweilzeit im dritten Rührbehälter betrug etwa 1 h, während der das Gemisch mit einem dreistufigen Kreuzbalkenrührer mit geringer Rührleistung (weniger als 0,1 W/I) gerührt wurde. Der Wasser und Alkohol enthaltende Brüden wurde abgezogen.

Das Produkt wurde gesammelt und über einen Vorlagebehälter einer Drucknutsche zugeführt und dort über ein Teflongewebe mit 10 µm Porenweite filtriert.

Man erhielt ein klares, von Katalysatorresten vollkommen freies Produkt mit einer Säurezahl von 0,01 mg KOH/g, einem Alkoholgehalt von 1,3 Gew.-% und einem Wassergehalt von 0,04 Gew.-%. Durch Strippen mit Wasserdampf konnte der Alkoholgehalt auf weniger als 0,01 Gew.-% vermindert werden.

### Vergleichsbeispiel

Das vorhergehende Beispiel wurde wiederholt, wobei jedoch der Rohester direkt in den ersten Rührbehälter geleitet wurde und die wässrige Natriumhydroxidlösung ebenfalls in den ersten Rührbehälter dosiert wurde.

Man erhielt ein Produkt mit einer Säurezahl von 0,08 mg KOH/g. Das Produkt weist gegenüber dem Beispiel 1 einen höheren Filterwiderstand auf.

## Patentansprüche

1. Verfahren zur Aufarbeitung eines rohen Esters einer Veresterungsreaktion, die mit einem metallhaltigen Veresterungskatalysator katalysiert ist, bei dem man
a) den rohen Ester bei einer Temperatur T von mehr als 100 °C unter einem Druck p, der gleich oder größer ist als der Dampfdruck von Wasser bei der Temperatur T, mit einer wässrigen Base versetzt,
b) das Ester-Base-Gemisch entspannt und Wasser abdampft,
c) die erhaltene flüssige Phase unter Bildung einer Wasser-in-Öl-Emulsion mit Wasser versetzt,
d) aus der Emulsion Wasser abdestilliert und
e) den Ester filtriert.

2. Verfahren nach Anspruch 1, wobei man den Schritt a) kontinuierlich durchführt, indem man die wässrige Base in einen Strom des rohen Esters einspritzt und den gemischten Strom durch einen statischen Mischer führt.

3. Verfahren nach Anspruch 1 oder 2, wobei man das Ester-Base-Gemisch eine Haltezeit von 15 Sekunden bis 10 Minuten beim Druck p hält.

4. Verfahren nach einem der vorhergehenden Ansprüche, wobei man mit der wässrigen Base 100 bis 300 % Neutralisationsäquivalente, bezogen auf die Säurezahl des rohen Esters, einbringt.

5. Verfahren nach einem der vorhergehenden Ansprüche, wobei die wässrige Base eine wässrige Alkalimetallhydroxidlösung ist.

6. Verfahren nach einem der vorhergehenden Ansprüche, wobei die Konzentration der wässrigen Base 0,5 bis 25 Gew.-% beträgt.

7. Verfahren nach einem der vorhergehenden Ansprüche, wobei man das Ester-Base-Gemisch im Schritt b) auf einen Druck von weniger als 800 mbar entspannt.

8. Verfahren nach einem der vorhergehenden Ansprüche, wobei man die im Schritt b) erhaltene flüssige Phase während einer Verweilzeit von 5 Minuten bis 1 Stunde mechanisch bewegt.

9. Verfahren nach einem der vorhergehenden Ansprüche, wobei man die flüssige Phase im Schritt c) mit 10 bis 60 g Wasser, bezogen auf 1 kg rohen Ester, versetzt.

10. Verfahren nach einem der vorhergehenden Ansprüche, wobei man im Schritt d) Wasser bei einer Temperatur von 60 bis weniger als 100 °C und einem Druck von weniger als 500 mbar abdestilliert.

11. Verfahren nach einem der vorhergehenden Ansprüche, wobei man im Schritt e) kein Filtrierhilfsmittel verwendet.

12. Verfahren nach einem der vorhergehenden Ansprüche, wobei man vor dem Schritt a) aus dem rohen Ester die Hauptmenge des nicht umgesetzten Alkohols entfernt.

13. Verfahren nach einem der vorhergehenden Ansprüche, wobei der metallhaltige Veresterungskatalysator unter Alkoholaten, Carboxylaten und Chelatverbindungen von Titan, Zirkonium, Zinn, Aluminium und Zink ausgewählt ist.

14. Verfahren nach einem der vorhergehenden Ansprüche, wobei die Veresterungsreaktion die Umsetzung einer unter aliphatischen Monocarbonsäuren mit wenigstens 5 Kohlenstoffatomen, aliphatischen C₄-C₁₀-Dicarbonsäuren, aromatischen Monocarbonsäuren, aromatischen Dicarbonsäuren, aromatischen Tricarbonsäuren, aromatischen Tetracarbonsäuren ausgewählten Carbonsäure und/oder Anhydriden davon umfasst.

15. Verfahren nach einem der vorhergehenden Ansprüche, wobei die Veresterungsreaktion die Umsetzung eines unter C₄-C₁₃-Alkoholen, Alkylenglykolmonoethern, Polyalkylenglykolmonoethem und Gemischen davon ausgewählten Alkohols umfasst.

## Claims

1. A process for working up a crude ester of an esterification reaction catalyzed by a metallic esterification catalyst, in which
a) the crude ester is admixed with an aqueous base at a temperature T of more than 100°C under a pressure p which is equal to or greater than the vapor pressure of water at the temperature T,
b) the ester-base mixture is decompressed and water is evaporated off,
c) the resulting liquid phase is admixed with water to form a water-in-oil emulsion,
d) water is distilled out of the emulsion and
e) the ester is filtered.

2. The process according to claim 1, wherein step a) is performed continuously, by spraying the aqueous base into a stream of the crude ester and conducting the mixed stream through a static mixer.

3. The process according to claim 1 or 2, wherein the ester-base mixture is kept at the pressure p for a hold time of from 15 seconds to 10 minutes.

4. The process according to any one of the preceding claims, wherein the aqueous base is used to introduce from 100 to 300% neutralization equivalents, based on the acid number of the crude ester.

5. The process according to any one of the preceding claims, wherein the aqueous base is an aqueous alkali metal hydroxide solution.

6. The process according to any one of the preceding claims, wherein the concentration of the aqueous base is from 0.5 to 25% by weight.

7. The process according to any one of the preceding claims, wherein the ester-base mixture is decompressed in step b) to a pressure of less than 800 mbar.

8. The process according to any one of the preceding claims, wherein the liquid phase obtained in step b) is moved mechanically over a residence time of from 5 minutes to 1 hour.

9. The process according to any one of the preceding claims, wherein the liquid phase in step c) is admixed with from 10 to 60 g of water based on 1 kg of crude ester.

10. The process according to any one of the preceding claims, wherein water is distilled off in step d) at a temperature of from 60 to less than 100°C and a pressure of less than 500 mbar.

11. The process according to any one of the preceding claims, wherein no filtration aid is used in step e).

12. The process according to any one of the preceding claims, wherein, before step a), the majority of the unconverted alcohol is removed from the crude ester.

13. The process according to any one of the preceding claims, wherein the metallic esterification catalyst is selected from alkoxides, carboxylates and chelate compounds of titanium, zirconium, tin, aluminum and zinc.

14. The process according to any one of the preceding claims, wherein the esterification reaction comprises the conversion of a carboxylic acid selected from aliphatic monocarboxylic acids having at least 5 carbon atoms, aliphatic C₄-C₁₀-dicarboxylic acids, aromatic monocarboxylic acids, aromatic dicarboxylic acids, aromatic tricarboxylic acids, aromatic tetracarboxylic acids and/or anhydrides thereof.

15. The process according to any one of the preceding claims, wherein the esterification reaction comprises the conversion of an alcohol selected from C₄-C₁₃-alcohols, alkylene glycol monoethers, polyalkylene glycol monoethers and mixtures thereof.

## Revendications

1. Procédé pour le traitement final d'un ester brut d'une réaction d'estérification qui est catalysée par un catalyseur d'estérification contenant un métal, dans lequel
a) on ajoute une base aqueuse à l'ester brut, à une température T de plus de 100 °C, sous une pression p qui est égale ou supérieure à la tension de vapeur de l'eau à la température T,
b) on détend le mélange ester-base et on fait évaporer l'eau,
c) on ajoute de l'eau à la phase liquide obtenue, avec formation d'une émulsion eau-dans-huile,
d) on élimine l'eau de l'émulsion par distillation et
e) on filtre l'ester.

2. Procédé selon la revendication 1, dans lequel on effectue en continu l'étape a), en injectant la base dans un courant de l'ester brut et en faisant passer le courant mixte dans un mélangeur statique.

3. Procédé selon la revendication 1 ou 2, dans lequel on maintient le mélange ester-base sous une pression p pendant un temps d'arrêt de 15 secondes à 10 minutes.

4. Procédé selon l'une quelconque des revendications précédentes, dans lequel on introduit avec la base aqueuse 100 à 300 % d'équivalents de neutralisation, par rapport à l'indice d'acide de l'ester brut.

5. Procédé selon l'une quelconque des revendications précédentes, dans lequel la base aqueuse est une solution aqueuse d'hydroxyde de métal alcalin.

6. Procédé selon l'une quelconque des revendications précédentes, dans lequel la concentration de la base aqueuse vaut de 0,5 à 25 % en poids.

7. Procédé selon l'une quelconque des revendications précédentes, dans lequel on détend le mélange ester-base dans l'étape b) jusqu'à une pression de moins de 800 mbars.

8. Procédé selon l'une quelconque des revendications précédentes, dans lequel on met la phase liquide obtenue dans l'étape b) en mouvement mécaniquement pendant un temps de séjour de 5 minutes à 1 heure.

9. Procédé selon l'une quelconque des revendications précédentes, dans lequel on ajoute à la phase liquide dans l'étape c) 10 à 60 g d'eau, par rapport à 1 kg d'ester brut.

10. Procédé selon l'une quelconque des revendications précédentes, dans lequel on élimine l'eau dans l'étape d) par distillation à une température de 60 à moins de 100 °C et sous une pression de moins de 500 mbars.

11. Procédé selon l'une quelconque des revendications précédentes, dans lequel on n'utilise aucun adjuvant de filtration dans l'étape e).

12. Procédé selon l'une quelconque des revendications précédentes, dans lequel avant l'étape a) on élimine de l'ester brut la quantité principale de l'alcool n'ayant pas réagi.

13. Procédé selon l'une quelconque des revendications précédentes, dans lequel le catalyseur d'estérification contenant un métal est choisi parmi des alcoolates, des carboxylates et des composés de type chélate de titane, zirconium, étain, aluminium et zinc.

14. Procédé selon l'une quelconque des revendications précédentes, dans lequel la réaction d'estérification comprend la réaction d'un acide carboxylique choisi parmi des acides monocarboxyliques aliphatiques ayant au moins 5 atomes de carbone, des acides dicarboxyliques aliphatiques en C₄-C₁₀, des acides monocarboxyliques aromatiques, des acides dicarboxyliques aromatiques, des acides tricarboxyliques aromatiques, des acides tétracarboxyliques aromatiques et/ou leurs anhydrides.

15. Procédé selon l'une quelconque des revendications précédentes, dans lequel la réaction d'estérification comprend l'estérification d'un alcool choisi parmi des alcools en C₄-C₁₃, des monoéthers d'alkylèneglycols, des monoéthers de polyalkylèneglycols et des mélanges de ceux-ci.
